# EUROPEAN PATENT APPLICATION

(11) **EP 3 878 840 A1**
(43) Date of publication of application: **15.09.2021**
(21) Application number: 21386019.0
(22) Date of filing: 05.03.2021
(51) Int. Cl.: C07C 403/20, C07D 207/404, A61K 31/164, A61P 17/00

(54) **CRYSTALLINE FORMS OF SALTS OF N1,N12-DI (ALL-TRANS-RETINOYL)SPERMINE, PROCESSES FOR THEIR PRODUCTION, AND PHARMACEUTICAL COMPOSITIONS THEREOF**

(30) Priority: 10.03.2020 GR 20200100131
(71) Applicant: University of Patras, 26504 Rio Achaia (GR); Papaioannou, Dionysios, 26221 Patras (GR); Drainas, Dionysios, 26504 Patras (GR); Avgoustakis, Konstantinos, 26442 Patras (GR)
(72) Inventor: Papaioannou, Dionysios, 26221 Patras (GR); Drainas, Dionysios, 26504 Patras (GR); Avgoustakis, Konstantinos, 26442 Patras (GR)

(57) **Abstract**

The present invention provides crystalline forms of salts of *N*¹*,N*¹²*-*di*(all-trans-*retinoyl)spermine and specifically of the dihydroxysuccinimidyl and the hydrochloride-hydroxysuccinimide salts of *N*¹,*N*¹²-*di*(*all-trans-*retinoyl)spermine, as well as pharmaceutical formulations for topical, parenteral and oral administration of said crystalline forms. It also provides processes for the production of the crystalline forms of the dihydroxysuccinimidyl salt and the mixed hydrochloride-hydroxysuccinimide salt of the *N*¹,*N*¹²-di*(all-trans*-retinoyl)spermine conjugate. Said crystalline forms are useful for the treatment of dermatological diseases such as psoriasis, inflammations and acne, as well as in cosmetics for example in anti-aging.

## Description

### TECHNICAL FIELD

The invention provides crystalline forms of salts of *N*¹,*N*¹²-di(*all-trans*-retinoyl)spermine and processes for producing them. These crystalline forms are useful for the treatment of dermatological diseases such as psoriasis, inflammation and acne, as well as for cosmetic use such as in anti-aging.

### BACKGROUND OF THE INVENTION

Recently, the synthesis of a wide variety of conjugates of naturally occurring polyamines, e.g. spermine, spermidine, and synthetic polyamines (linear, branched and cyclic), with retinoid acids (e.g. *all-trans*-retinoic acid (RA) and lower homologs, 9- and 13-cis-retinoic acid, acitretin) has been described. These conjugates exhibited stronger inhibition of RNase P, derived from *D. discoideum* and human epidermal keratinocytes, relative to the parent retinoids or the simple combination of the corresponding free polyamines and retinoids. The same studies showed that the potency of inhibition was related to the number of (a) the free amino groups and (b) the retinoid structures in the conjugate. In addition, these conjugates also showed *in vitro* inhibitory activity on the production of interleukin-2 (IL-2) and interferon-γ (IFN-γ) from peripheral - blood mononuclear cells of healthy human samples. The results indicated that these conjugates have important potential clinical application in the treatment of keratinization abnormalities and inflammation [WO 2004018001 A1; Magoulas et al, Eur Med. Chem., 44:2689-2695 (2009)].

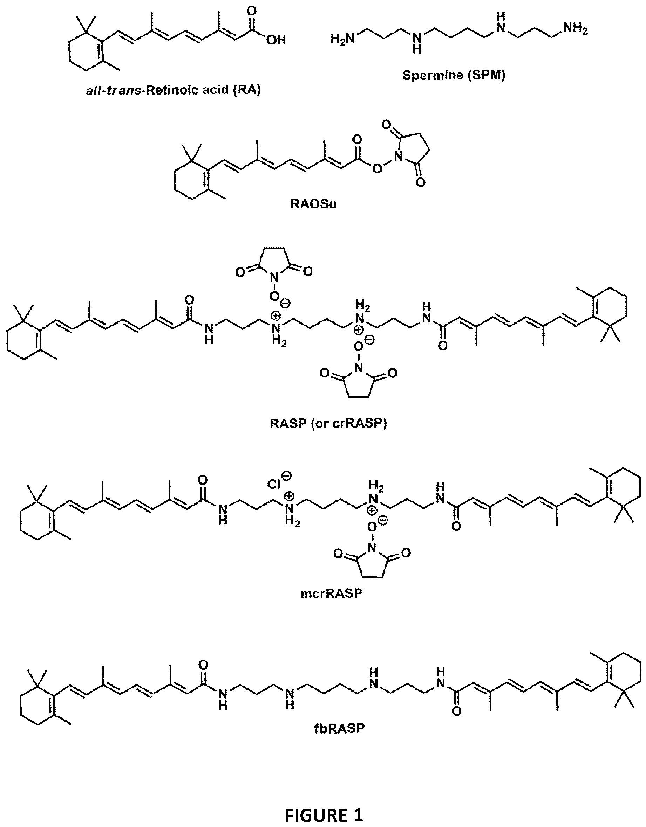

The most interesting of these conjugates, *N*¹,*N*¹²-di(*all-trans*-retinoyl)spermine, in the form of its dihydroxysuccinimidyl salt (abbreviated herein as RASP, Figure 1), inhibits angiogenesis in chorioallantoic membrane of chicken embryo, has anti-cancer activity on prostate cancer cells, as well as antioxidant and anti-inflammatory activity *in vitro* and *in vivo,* while showing neither toxicity nor teratogenicity (a typical side effect of the retinoids used in current therapeutic) in a reproductive study in two generations of rats.

The inventors have recently shown that RASP increases expression of the transcription factor TGF-β, thus inhibiting hyperproliferation and abnormal differentiation of epidermal keratinocytes (hyperproliferation and aberrant differentiation of the epidermal keratinocytes is characteristic of psoriasis), and inhibits the expression of collagenases, which results in the protection of collagen from degradation (destruction of collagen is characteristic of the aging of epidermis). It also increases the expression of TNF (tumor necrosis factor) and its receptors, and the interaction of its signaling through a p53-independent increase of p21, which inhibits cell cycle progression and induces apoptosis (inhibition of apoptosis is characteristic of both psoriasis and aging). This results in a decrease in the number of keratinocytes (psoriasis) as well as in the aged skin cells (aging). Also RASP inhibits angiogenesis (see above). Increase in angiogenesis is observed in both psoriasis and skin aging. Furthermore, it inhibits significantly IL-2 and IFN-γ production by CD4⁺ and CD8⁺ cells. IL-2 and INF-γ are cytocines of Th1 type which promote inflammation and are involved in the pathogenesis of psoriasis. These results show that RASP has potentially potent antipsoriatic and anti-aging effect (renewal of epidermis occurs through the removal of senescent cells). For the same reasons, RASP has potent action in the treatment of acne (acne is characterized by increased sebum production, abnormal keratinization of the follicular infundibulum, mediated responses to the bacterium *Propionibacterium acnes,* and inflammation).

The synthesis of these conjugates is achieved by condensing the free polyamines (e.g., spermine, Spm, Figure 1) with the isolable, crystalline, succinimididyl esters of the retinoids acids, such as the succinimidyl ester of *all-trans*-retinoic acid (RAOSu, Figure 1). The afore mentioned succinimidyl esters are readily prepared by the condensation of retinoid acids (e.g., the *all-trans-*retinoic acid, RA, Figure 1) with *N*-hydroxysuccinimide (HOSu) in the presence of a dehydrating agent, such as e.g. the *N*,*N'-*dicyclohexylcarbodiimide (DCC). The same process is used for the production of *N*¹*,N*¹²-di(*all-trans*-retinoyl)spermine, which is obtained in the form of the corresponding disalt (RASP) with *N*-hydroxysuccinimide, which is co-produced in the condensation reaction. RASP is received from the reaction mixture, by a simple filtration, mainly as an amorphous yellow solid [WO 2004018001 A1; Magoulas et al, Eur Med. Chem., 44:2689-2695 (2009)].

The thus produced product (RASP) is usually accompanied by a small and variable (depending on reaction scale) amount of *N*¹-(*all-trans*-retinoyl)spermine, in the form of the trishydroxysuccinimide salt thereof, derived from the incomplete diacylation of spermine, but can be further purified, if necessary, by flash column chromatography (FCC). In addition, the fine granular characteristics of the product can cause problems, in particular for large-scale preparations, due to long-lasting vacuum filtration of the reaction mixture. This results in a slight surface oxidation due to the simultaneous evaporation of the volatile reaction solvent (dichloromethane) and in a consequent varying coloration (deep yellow to brown) of the various batches of the obtained product.

Thus, it becomes apparent that there is a need for (a) the preparation of a RASP product of stable composition and of the highest possible purity, free of the by-product *N*¹-(*all-trans*-retinoyl)spermine without resorting to costly chromatographic methods, and in such form (e.g. crystalline form) that facilitates isolation without the aforementioned problems, particularly during the preparation of the product RASP in large scale, or (b) the preparation of alternative, possibly crystalline, salts of *N*¹,*N*¹²-*di*(all-trans-retinoyl)spermine with pharmaceutically acceptable acids, e.g. of its dihydrochloride salt. The present invention addresses all these requirements.

### SUMMARY OF THE INVENTION

The above problems led the present inventors to explore the possibility of producing RASP in alternative forms, for example in pure crystalline form.

Thus, the present invention provides processes for the production of a crystalline dihydroxysuccinimidyl salt of *N*¹,*N*¹²-*di*(all-trans-retinoyl)spermine.

A crystalline form of the crystalline dihydroxysuccinimidyl salt of *N*¹,*N*¹²-*di*(all-trans-retinoyl)spermine is also provided which is characterized from a powder X ray diffraction spectrum (XRD) as described in the present invention.

According to another embodiment, the present invention provides processes for the production of a crystalline mixed hydrochloride-hydroxysuccinimidyl salt of *N*¹*,N*¹²-*di*(all-trans-retinoyl)spermine.

Another embodiment is directed towards the crystalline form of a mixed hydrochloride-hydroxysuccinimidyl salt of *N*¹,*N*¹²-*di*(all-trans-retinoyl)spermine which is characterized from a powder X ray diffraction spectrum (XRD) as described in the present invention.

The crystalline salts of the present invention are useful in medicine. According to certain preferred embodiments, the afore mentioned crystalline forms are useful in the treatment of dermatological diseases, such as psoriasis, skin inflammation and acne.

This invention also provides pharmaceutical compositions for the treatment of dermatological diseases caused by abnormal keratinization, such as psoriasis, as well as for the treatment of skin inflammation and acne.

Also provided are cosmetic compositions for use on the skin and especially for the improvement of unwanted skin conditions.

Other aspects and further areas of application of this invention will be apparent from the detailed description which follows.

### BRIEF DESCRIPTION OF THE DRAWINGS

The present invention will now be described with reference to certain embodiments thereof illustrated in the accompanying drawings. It should be noted that the accompanying drawings illustrate preferred embodiments of the invention, and therefore they should not be construed as limiting the scope of the invention.
Figure 1 illustrates the structures of *all*-*trans*-retinoic acid, its active ester RAOSu with HOSu, spermine, the salts RASP/crRASP and mcrRASP and *N*¹,*N*¹²-*di(all-trans*-retinoyl)spermine (fbRASP).
Figure 2 shows the HPLC chromatogram of the salt RASP.
Figure 3 shows the XRD spectrum of RASP.
Figure 4 shows in A) the HPLC chromatogram of the crRASP salt and B) the ESI-MS spectrum of the salt crRASP.
Figure 5 presents the 600 MHz ¹H-NMR spectrum of the salt crRASP.
Figure 6 presents in A) the 150 MHz ¹³C-NMR spectrum of salt crRASP, as well as regions of the spectrum of Figure 6A with B) δ= 177 - 120 ppm and C) δ= 52 - 10 ppm.
Figure 7 presents the XRD spectrum of the salt crRASP.
Figure 8 presents the HPLC chromatograms A) of the crystalline product formed from the reaction of spermine (Spm) and RAOSu using methanol as the solvent and B) of the filtrate of the crystalline product formed from the reaction of Spm and RAOSu using methanol as the solvent.
Figure 9 shows the HPLC chromatogram of salt crRASP from the reaction of spermine and RAOSu using chloroform as the solvent and crystallization from methanol.
Figure 10 shows an HPLC chromatogram of the mixed salt mcrRASP.
Figure 11 shows the 600 MHz ¹H-NMR spectrum of the mixed salt mcrRASP.
Figure 12 shows in A) the 150 MHz ¹³C-NMR spectrum of the mixed salt mcrRASP, as well as regions of the spectrum of Figure 12A with B) δ = 178 - 118 ppm and C) δ = 52 - 10 ppm.
Figure 13 presents the XRD spectrum of the mixed salt mcrRASP.

### DETAILED DESCRIPTION OF THE INVENTION

For terms for which no particular definition is provided in the present description, meanings should be assigned which would have been given by a person skilled in the art in the light of the present disclosure and of its more general context.

The problems appearing in the production of RASP according to the prior art, led the present inventors to explore the possibility of producing RASP in alternative forms, for example in pure crystalline form. After extensive studies, involving a wide variety of solvents, mixtures thereof and crystallization conditions, it was not possible to crystallize the product RASP. Thus, the possibility of converting the investigated RASP to other pharmaceutically acceptable salts, such as for example the dihydrochloride salt, was explored. Given the vulnerability of retinoids to strong acids such as hydrogen chloride, the conversion of RASP to its corresponding dihydrochloride salt using ammonium chloride was investigated. After extensive studies, the inventors have unexpectedly discovered that RASP can be directly converted to the corresponding crystalline mixed hydrochloride-hydroxysuccinimide salt (abbreviated herein as mcrRASP) but not to the corresponding dihydrochloride salt thereof, with ammonium chloride in boiling methanol and then overnight cooling of the reaction solution to 4-8 °C. The latter could not be either obtained from the free base RASP (abbreviated herein as fbRASP), which is produced in pure form as described in the prior art [WO 2004018001 A1; Magoulas et al, Eur Med. Chem., 44:2689-2695 (2009)]. Given the above, the inventors have repeated their attempts towards crystallization of RASP, this time using specially methanol as the solvent. Indeed, dissolution of the crude product RASP, as obtained by the reaction of RAOSu with Spm in the solvent CH₂Cl₂ [WO 2004018001 A1; Magoulas et al, Eur Med. Chem., 44:2689-2695 (2009)], in boiling methanol and then overnight cooling, yielded pure crystalline RASP (abbreviated herein as crRASP).

Therefore, an aspect of the present invention is to provide a process for the production of the crystalline dihydroxysuccinimidyl salt of *N*¹,*N*¹²-*di*(all-trans-retinoyl)spermine, which comprises the step of crystallization in the presence of a solvent.

According to an embodiment, said crystallization solvent is an alcohol with a boiling point lower than 100 °C, preferably methanol. RASP is mixed with an alcohol solvent with a boiling point lower than 100 °C, preferably methanol, and the mixture is boiled under reflux until complete dissolution. RASP can be produced using any method known in the art, for example in the form of a fine-grained yellow solid by the method which has been described [WO 2004018001 A1; Magoulas al, Eur Med. Chem., 44:2689-2695 (2009)].

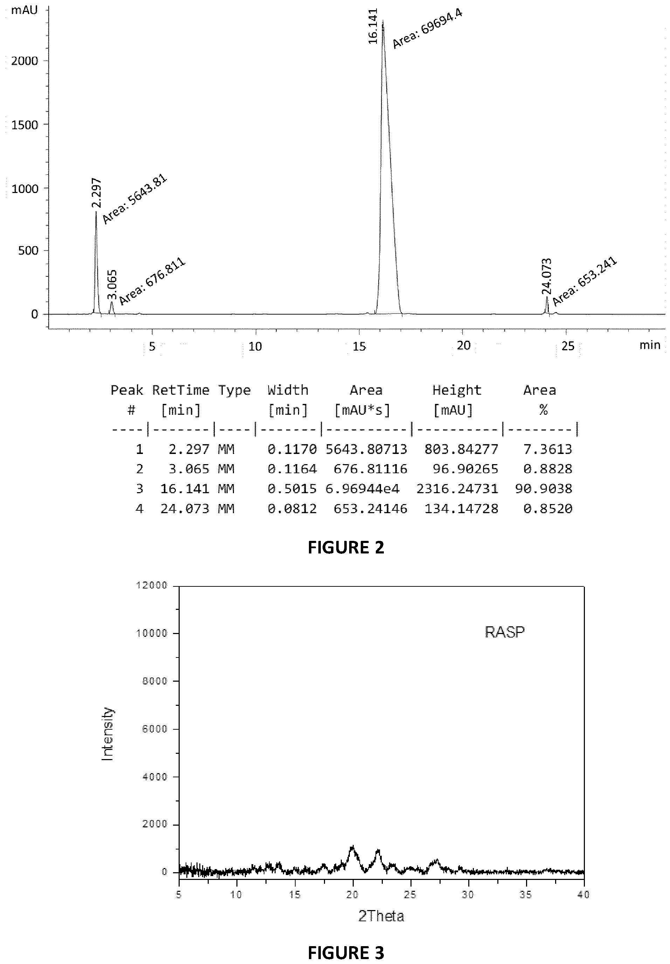

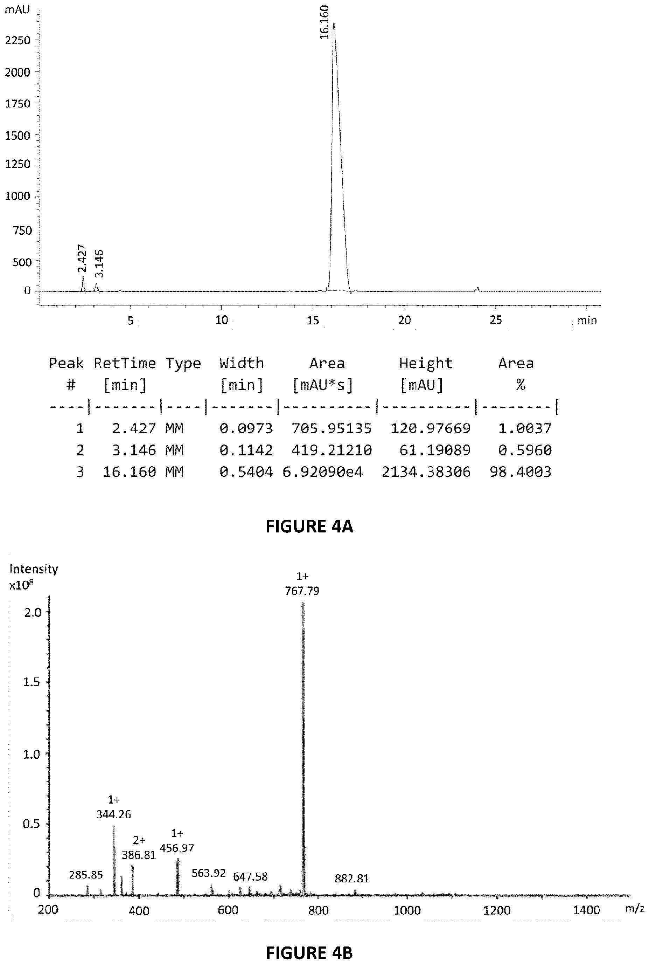

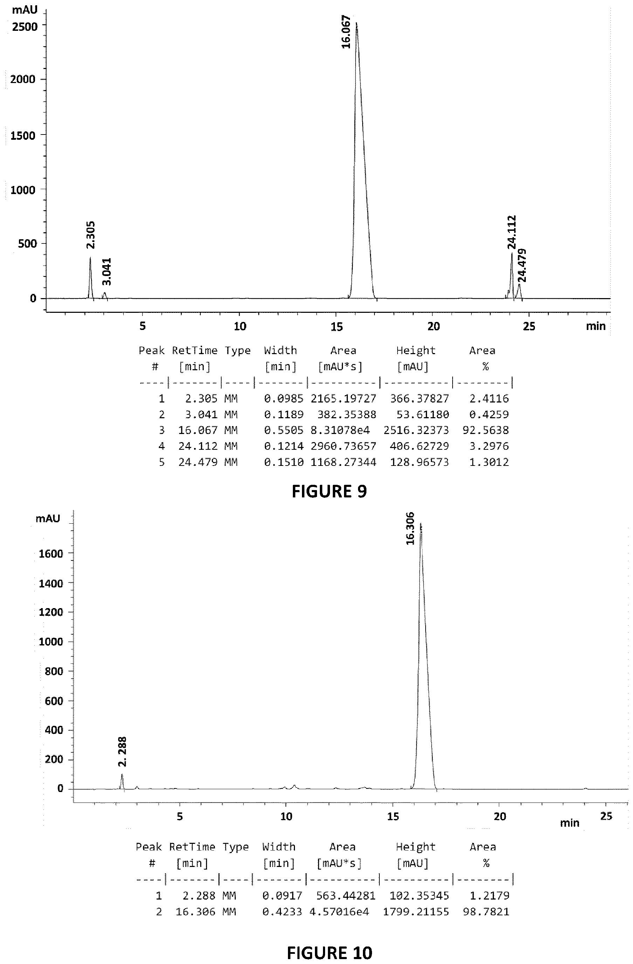

The solution is allowed to come to room temperature and then refrigerated (4-8 °C) overnight. The thus obtained crystalline solid (abbreviated herein as crRASP) can be obtained using a suitable method known in the prior art. For example, pure crRASP can be obtained by vacuum filtration, filtering the solid on the filter with ice-cold methanol and diethyl ether and finally vacuum drying over a suitable drying agent. From Figures 2 and 4 and studying the ESI-MS spectra of the various fractions, it occurs that RASP (retention time: ca 16.1 min) is obtained with a purity of 90.9% along with 7.4% of the mono-retinoylated spermine (retention time: ca 2.3 min). The remainder presumably consists of isomeric mono-retinoylated spermine (retention time: ca 3.1 min) and tri-retinoylated spermine (retention time: ca 24.1 min). Crystallization of RASP using methanol, nevertheless, leads to product (crRASP) with greater than 98% purity (Figure 10).

In another preferred embodiment of the invention, spermine (Spm) reacts with almost twice the molar amount of RAOSu in freshly distilled anhydrous chloroform (CHCl₃), as described in the prior art [WO 2004018001 A1; Magoulas et al, Eur Med. Chem., 44:2689-2695 (2009)] for the production of RASP. After completion of the reaction, the solvent was evaporated under vacuum in a rotary evaporator and the resulting residue was dissolved with heating in the minimum volume of an alcohol with a boiling point lower than 100 °C, preferably methanol. Overnight cooling results in precipitation of the product, whereby crRASP is obtained by vacuum filtration, filtering the solid on the filter with ice-cold methanol and diethyl ether and finally vacuum drying over a suitable drying agent. The HPLC chromatogram of the product is provided in Figure 9 indicating that the conjugate crRASP is obtained with 92.6% purity.

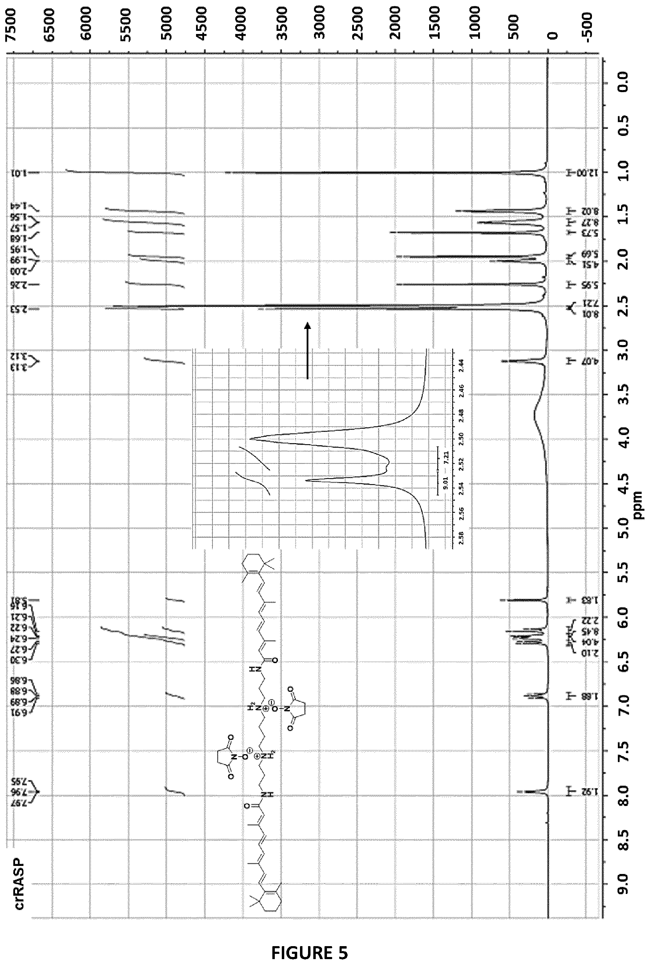

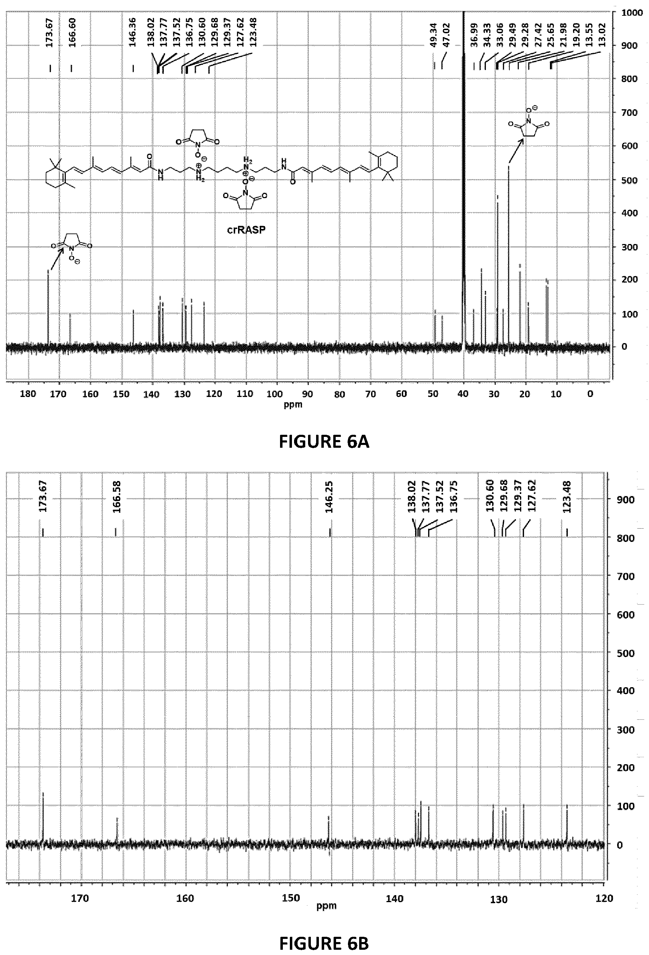

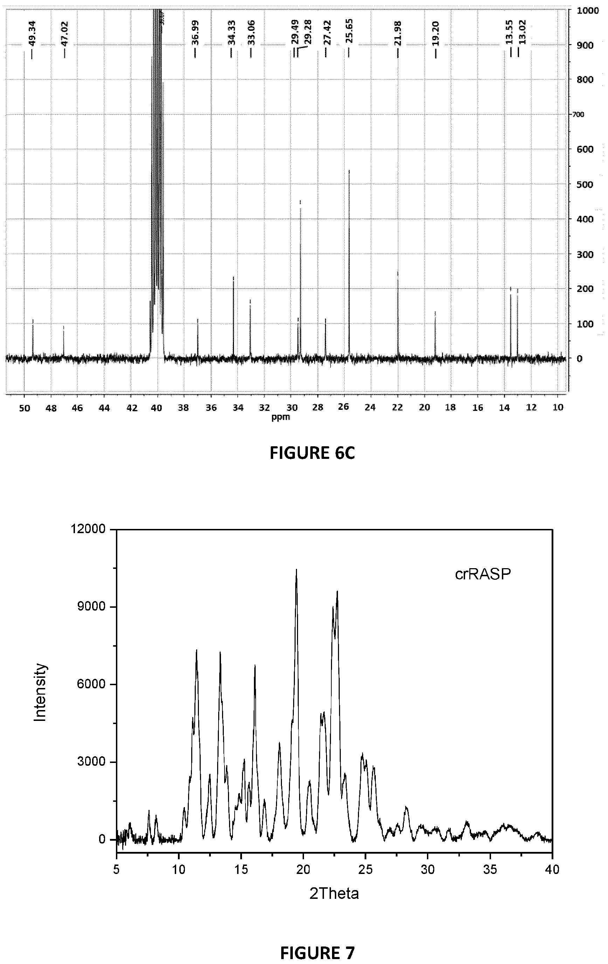

According to another aspect of the present invention, a crystalline form of the dihydroxysuccinimidyl salt of *N*¹,*N*¹²-*di*(*all-trans*-retinoyl)spermine (crRASP) is provided, which is characterized by a powder X-ray diffraction spectrum (XRD) with main peaks at 11.43, 13.33, 16.11, 19.44, 22.40 and 22.72 ±0.20 degrees 2θ, substantially as illustrated in Figure 7. An HPLC chromatogram of the crystalline salt crRASP, as well as ESI-MS, ¹H- and ¹³C-NMR and X-ray diffraction (XRD) spectra are shown in Figures 4A, 4B, 5, 6A (also 6B and 6C) and 7, respectively. For comparison, a representative HPLC chromatogram and an XRD spectrum of the salt RASP are provided in Figures 2 and 3, respectively.

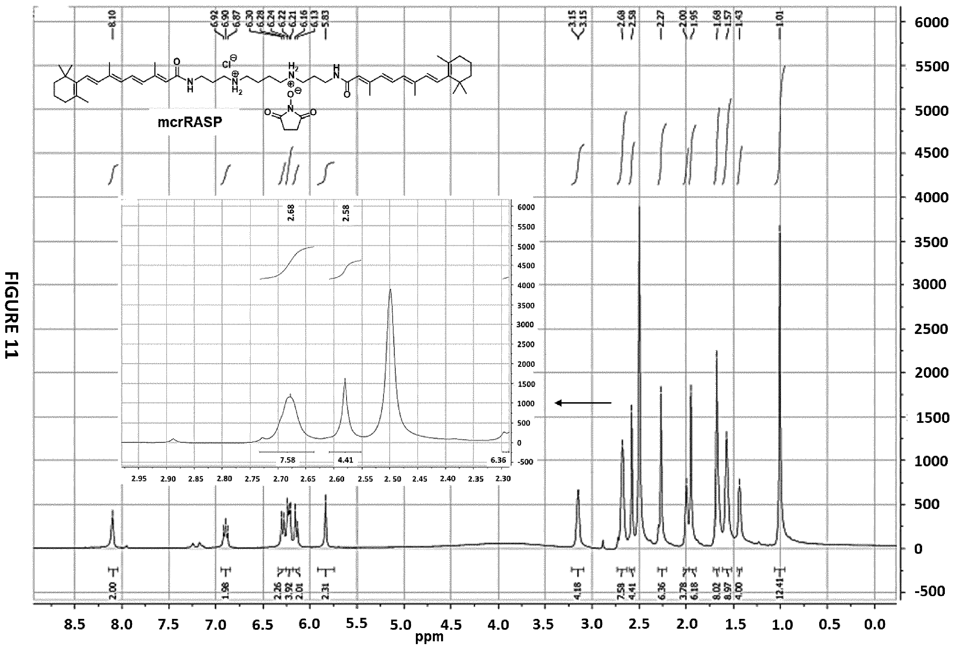

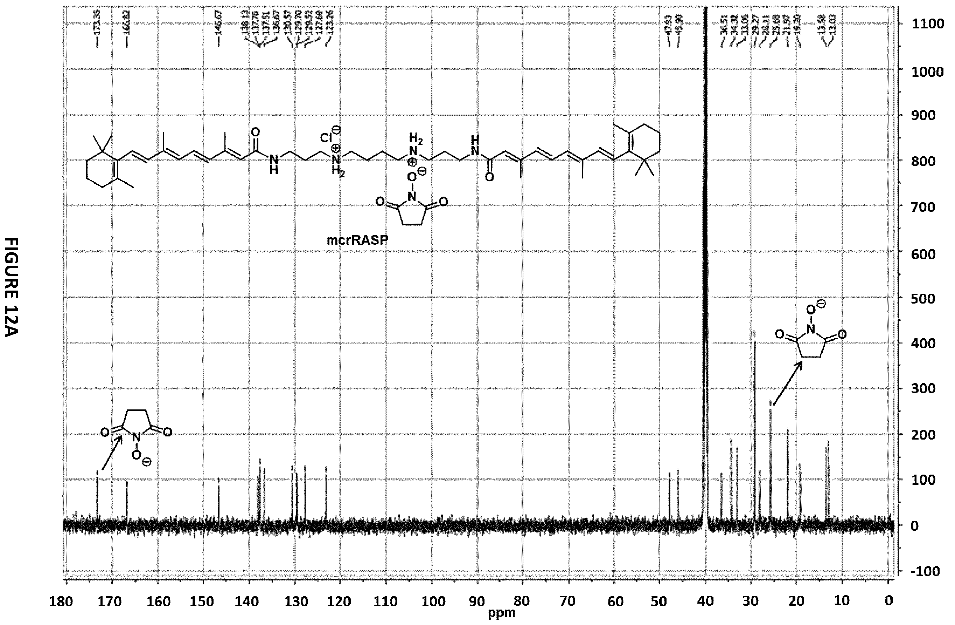

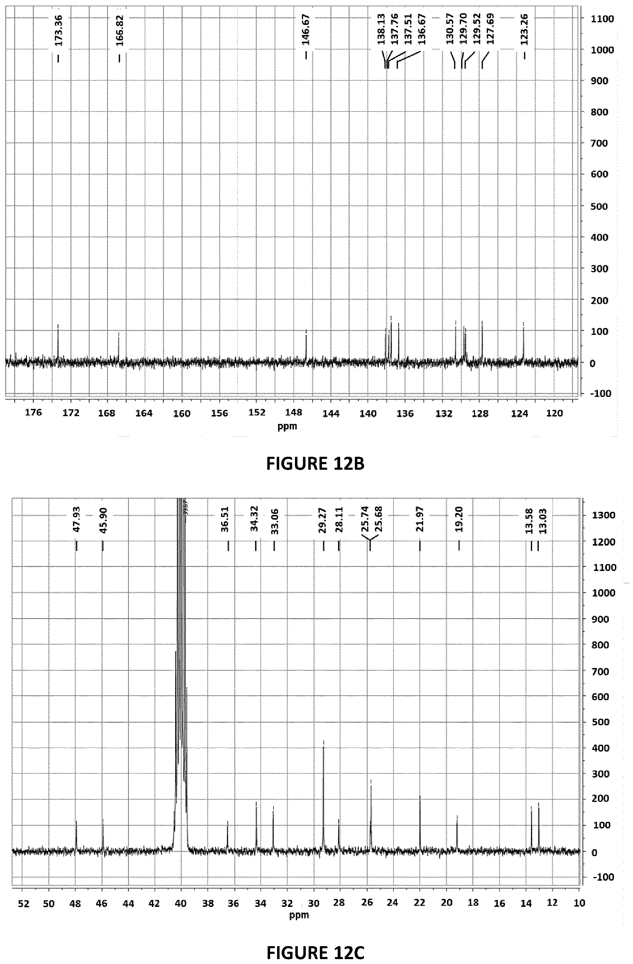

In another preferred embodiment of the invention, a process for the preparation of the crystalline mixed hydrochloride-hydroxysuccinimide salt of *N*¹*,N*¹²*-di(all-trans-*retinoyl)spermine (mcrRASP) is provided. The process involves as the first step the reaction of RASP with ammonium chloride (NH₄Cl) in an organic solvent. The solvent is an alcohol with a boiling point lower than 100 °C, preferably methanol. The second step of the process comprises of the direct crystallization of mcrRASP from the solvent. For example, RASP or crRASP and ammonium chloride (NH₄Cl) in a molar ratio of 1: 2.2 are taken in methanol, and the resulting mixture is brought to a vigorous reflux for 30 min. The thus obtained yellow solution is cooled at 4-8 °C overnight to precipitate the mcrRASP, which is obtained by vacuum filtration, washing with cold methanol and diethyl ether and finally drying under vacuum. The HPLC chromatogram of the product (Figure 10) shows a very high purity (*ca* 98.8%). The ¹H-NMR spectrum of the compound (Figure 11) verifies the loss of one of the two *N*-hydroxysuccinimides. The ¹³C-NMR and XRD spectra of the mcrRASP product are provided in Figures 12A (and 12B and 12C) and 13, respectively. It should be noted that prolonged heating or heating with excess NH₄Cl does not cause any further change or results in the appearance of a product with a more complex ¹H-NMR spectrum. Similar results (complex ¹H-NMR spectrum and HPLC chromatogram) were obtained during an attempt to prepare the corresponding dihydrochloride salt from pure fbRASP (Figure 1) and NH₄Cl in the ratio 1: 2.2 using methanol as the solvent, after vigorous reflux for 30 min.

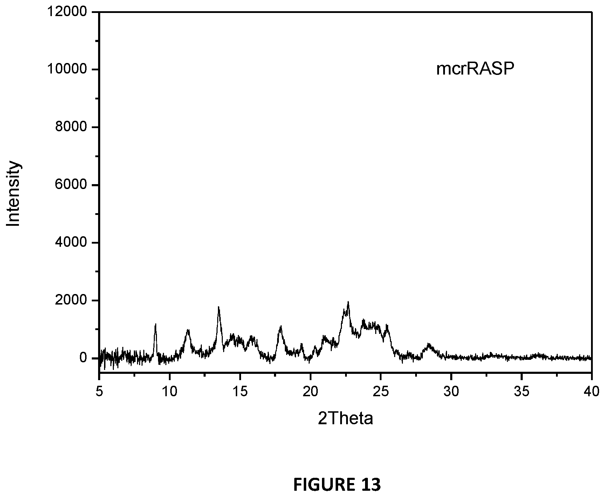

A further embodiment is directed to a crystalline form of the mixed hydrochloride-hydroxysuccinimide salt of *N*¹,*N*¹²-di(*all-trans*-retinoyl)spermine (mcrRASP), which is characterized by a powder X-ray diffraction spectrum (XRD) with main peaks at 9.02, 11.29, 11.39, 13.49, 17.92, 22.36, 22.66, 23.00, 23.62, 23.76, 23.84, 24.42, 24.58 and 25.38 ±0.20 degrees 2θ, substantially as illustrated in Figure 13. An HPLC chromatogram of the crystalline salt mcrRASP, as well as ¹H- and ¹³C-NMR and X-ray diffraction (XRD) spectra are shown in Figures 10, 11 and 12, respectively.

In a further embodiment, crystalline forms of the dihydroxysuccinimidyl salt of *N*¹,*N*¹²-di(*all*-*trans*-retinoyl)spermine (crRASP) or the mixed hydrochloride-hydroxysuccinimide salt of *N*¹,*N*¹²-di(*all-trans*-retinoyl)spermine (mcrRASP) of the present invention are provided for use in medicine. In particular, the use of the above crystalline forms is indicated for use in diseases, syndromes or symptoms associated with increased or uncontrolled cell proliferation and/or activation of the inflammatory response. Examples of such diseases, syndromes or symptoms include abnormalities of keratinization, skin inflammation, acne or other adverse skin condition. Examples of abnormalities of keratinization include ichthyosis, psoriasis, atopic dermatitis, acne rosacea, and palmoplantar keratosis. This adverse skin condition may include, but is not limited to, eczema such as seborrheic eczema, skin rashes, dry or scaly skin to name a few.

The invention also provides pharmaceutical compositions which comprise the crystalline forms of the dihydroxysuccinimidyl salt of *N*¹,*N*¹²-di(*all-trans*-retinoyl)spermine (crRASP) or the mixed hydrochloride-hydroxysuccinimide salt of *N*¹,*N*¹²-di(*all-trans-*retinoyl)spermine (mcrRASP) which are claimed herein, and at least one pharmaceutically acceptable carrier, adjuvant or excipient. These substances that can be added to therapeutic agents are known to the expert and include preservatives, wetting agents, emulsifying agents, buffering agents for pH adjustment, stabilizers, suspending agents, detergents, antioxidants, oleaginous carriers, hydrocarbon bases, fragrances, phospholipids, diluents (fillers), disintegrants, binders, lubricants, sweeteners, colorants, thickening agents, solvents, solubilizers, plasticizers, agents for the creation of prolonged release tablets, salts for osmotic pressure changes, coating polymers and other agents.

The aforementioned pharmaceutical compositions are useful in medicine. In particular, the use of the above crystalline forms is indicated for use in diseases, syndromes or symptoms associated with increased or uncontrolled cell proliferation and / or activation of the inflammatory response. Examples of such diseases, syndromes or symptoms include abnormalities of keratinization, skin inflammation, acne or other adverse skin condition. Examples of abnormalities of keratinization include ichthyoses, psoriasis, atopic dermatitis, acne rosacea, and palmoplantar keratosis. This adverse skin condition may include, but is not limited to, eczema such as seborrheic eczema, skin rashes, dry or scaly skin to name a few.

The formulation of the specific composition is generally dependent on the particular indication and the route of administration to a patient. The preparations described in the present invention are formulated for administration to the subject via any conventional means including but not limited to parenteral, transdermal, oral and topical administration. The term "parenteral" as used herein includes subcutaneous, intravenous, intraarterial, intramuscular, intraperitoneal, intrathecal, intraventricular, intrasternal, intracranial, and intraosseous injection and infusion techniques. For example, pharmaceutical preparations may be administered parenterally such as intravenously, intramuscularly or subcutaneously in the form of injectable or infusion solutions, microcapsules or (intramuscular or subcutaneous) implants.

Parenteral dosage forms can be administered in the form of sterile or sterilizable injectable preparations such as injectable solutions, suspensions, dry or lyophilized products ready for dissolution or suspension in pharmaceutically acceptable carriers for injection (reconstitution powders), and emulsions. Among the acceptable carriers and solvents that could be employed are water, dextrose, the Ringer solution , isotonic sodium chloride, vehicles that are miscible with water such as, without limitation of the range of options, ethyl alcohol, propylene glycol, polyethylene glycol, polypropyleneglycol and nonaqueous vehicles such as, without limitation of the range of options, vegetable oils (e.g., corn oil, cottonseed oil, sesame oil), injectable organic esters such as oleyl acetate, isopropyl myristate and benzyl benzoate.

The administration can also be carried out orally, for example in the form of pills, tablets, coated tablets, enteric tablets, granules, pellets, hard and soft gelatin capsules, solutions, syrups, emulsions, or suspensions. Preparations for oral administration in solid or liquid form can be produced by any method known in the manufacture of pharmaceutical preparations and such preparations may contain one or more agents selected from the groups comprising diluents, binders, disintegrants, lubricants, solvents, as well as sweeteners, sweetening agents and colorants in order to provide palatable and elegant formulations. Suitable excipients may be for example, inert diluents such as microcrystalline cellulose, cellulose derivatives, starch and derivatives thereof, the calcium carbonate, the sodium carbonate, the lactose, the calcium phosphate or sodium phosphate, granulating and disintegrating agents, as well as other non-toxic compatible fillers, fillers, nonionic, cationic, anionic or ampholytic surface active agents, buffers, antioxidants, lubricants, preservatives or stabilizers and other agents which are routinely used in pharmaceutical forms. Tablets may be non-coated media or may be coated with film or sucrose by known techniques. In some cases, coatings may be produced by known techniques to delay dissolution and prolong absorption from the gastrointestinal tract and thus provide constant activity over long periods of time. For example, materials for prolonged action are pH sensitive and not polymers and oily and waxy substances such as the monoglycerides or diglycerides of stearic acid. Suitable excipients for the production of solutions or suspensions or syrups are for example water, saline, alcohols, glycerol, polyols, sucrose, inverted sugar, glucose, vegetable oils, suspending agents and others. Suitable excipients for microcapsules or implants are for example the copolymers of glycolic and of lactic acid.

Topical formulations include aqueous or non-aqueous preparations such as, for example, solution, aerosol, cream, ointment, gel, paste or lotion. Suitable bases for topical semi-solid and liquid formulations are for instance fatty alcohols, fatty acids, fatty alcohol esters, lanolin, petrolatum, paraffin, polyethylene glycol, emulsifiers, antioxidants, permeation enhancers, and oily carriers such as vegetable and mineral oils. In transdermal delivery systems belong the pharmaceutical forms bandage, patch or membrane and, similarly to the topical formulations, may include substances that enhance penetration so as to assist the transfer of the active substances through the tissue. Such permeation enhancers include but not limited to , acetone, various alcohols such as ethanol, isopropanol, decanol, oleyl alcohol, tetrahydrofuranol, dipropylene glycol, propylene glycol, 1,2 butylene glycol, 1,3 butylene glycol, urea, alkylosulfoxide as dimethylsulfoxide, pyrrolidones such as polyvinyl pyrrolidone, dimethylacetamide, dimethylformamide, polyethyleneglycol, Kollidon (povidone, polyvidone), Azone, Transcutol and surface active agents such as polysorbate 80, sodium lauryl sulfate, 2-dodekyloxyethanol (Brij 36T), sorbitan monostearate and terpenes. The transdermal patches are typically composed of fiber or paper impregnated with an appropriate dose of medicament for transdermal administration.

The pharmaceutical preparations typically contain from about 0.5% to 90% by weight of the crystalline salts of the present invention. The amount of the crystalline salts in the pharmaceutical composition is typically between 0.5 of mg to 1000 mg, preferably from about 1 mg to about 500 mg.

The pharmaceutical composition is administered to the patient at a therapeutically effective dose or at a prophylactically effective dose. A "therapeutically effective dose" of a pharmaceutical composition is an amount sufficient to stop, reverse or reduce the progression of the disorder. A "prophylactically effective dose" of a pharmaceutical composition is an amount sufficient to prevent the onset of a disorder, for example eliminate, improve and/or delay the onset of the disorder. The dose can vary within wide limits and, as is customary and is known to the physician, will be adapted to the specific conditions in each individually present case. The dose depends, for example, on the particular compound employed, on the nature and severity of the disease to be treated, the manner and schedule of administration, or whether it is used for acute or chronic disease or as prophylaxis. The appropriate dosage can be established using clinical approaches well known in the medical art. in general, the daily dose for achieving the desired results in an adult weighing about 75 kg is from 0.01 mg/kg to 100 mg/kg, preferably from 0.1 mg/kg to 50 mg/kg, specifically from 0.1 mg/kg to 10 mg/kg (in each case in mg per kg of body weight). The daily dose can be divided, especially in the case of relatively large doses, into several, for example 2, 3 or 4 separate doses. Usually, depending on the response of each patient, it may be necessary to deviate upwards or downwards from the indicated daily dose.

The compounds of the invention are particularly suitable for cosmetic use. Preferably, these compounds are used to maintain good skin condition and/or for anti-aging. The afore mentioned desirable effects include, but not limited to, the treatment, reduction, or/and prevention of thin lines or wrinkles, the enhancement of pre-colllagen and collagen production, the improvement of skin barrier function, the improvement of skin hydration, the improvement of skin elasticity and strength, the prevention and/or treatment of skin atrophy, among others. According to this embodiment, the compositions can be formulated in all the pharmaceutical forms used normally for topical application, specifically in the form of aqueous, aqueous / alcoholic or oily solutions, in the form of suspensions or dispersions of lotion type or serum type, in the form of anhydrous or lipophilic gel, in the form of ointment, in the form of emulsions of liquid or semi-solid form which are obtained by dispersing a fatty phase in an aqueous phase (oil-in-water) or the reverse (water in oil), or suspensions or emulsions with flowable, semi-solid or solid consistency of cream-type or gel, or alternatively in the form of microemulsions, microcapsules or microparticles, or as dispersions of vesicles of ionic and/or non-ionic type. These compositions are formulated according to conventional techniques.

The compounds can also be used for hair in the form of aqueous, alcoholic or aqueous/ alcoholic solutions or in the form of creams, gels, emulsions or foams or alternatively in the form of aerosol compositions which also contain propellant gas under presssure.

The cosmetic compositions of the invention may also contain additives and adjuvants which are frequently used in pharmaceutical products, cosmetics, or dermatological products, such as petrolatum, paraffin, vegetable or mineral oils, lanolin alcohols and lanolin esters, fatty alcohols, fatty acids, fatty alcohol esters, moisturizing agents, emollients, emulsifiers, deodorant and antiperspirant agents, sunscreens, hydrophilic or lipophilic gelling agents, preservatives, antioxidants, solvents, fragrances, fillers, protective agents, bactericides, odor absorbants, colorants and dyes. The amounts of these various additives and adjuvants are those usually employed in the cosmetic field.

In a preferred embodiment, the concentration of the compounds according to the invention in the composition is between 0.001% and 10% by weight. Preferably, said concentration in the composition is between 0.01% and 4%, most preferably from 0.1% to 2% by weight.

### EXAMPLES

Hereinafter, examples are provided for a more detailed description of the invention. It is obvious that these examples are provided for illustrative purposes only and should not be construed as limiting the scope of the present invention to those cases described.

### EXAMPLE 1

### General experimental

Melting points were obtained on a Büchi SMP-20 apparatus and are not corrected. The ¹H-NMR and ¹³C-NMR spectra were obtained at 600.13 and 150.91 MHz, using a Bruker ASCEND 600 spectrometer in the solvent d6-DMSO. Electron-Spray Ionization (ESI) spectra were obtained using a BRUKER AMAZON SL spectrometer for solutions of the compounds in methanol. Elemental analyses were performed on a Carlo Erba EA 1108 elemental analyzer. All experiments were conducted with protection from light and under an argon atmosphere.

Reverse Phase High Performance Liquid Chromatography (RP-HPLC) was performed on the system of Agilent Technologies 1260 Afinity, Quaternary Pump VL, with Microsoft Windows 7 operating system and diode array ultraviolet detector Agilent 1260 DAD VL. The purity of the final products was determined using ultraviolet detection at 360 nm. The column used was the Agilent ZORBAX Eclipse Plus C 18 (100 x 4.6 mm) with a 3.5 µm particle diameter. The analyses were performed using elution solvents with varying composition and flow rate 1 mL/min. The eluents used were: A) trifluoroacetic acid (TFA) solution in H₂O 0.08% (v/v) and B) TFA solution in acetonitrile (ACN) 0.08% (v/v). Gradient elution from 50% to 100% B for 20 minutes and then 100% B for 20 to 30 minutes. Injection volume 20 mL. The sample was prepared by dissolving in DMSO and then adding 4 drops of TFA.

X-ray powder diffraction spectra (XRD) were performed on the BrukerAXSD8 Advance diffractometer, which employs Bragg-Brentano geometry and has a LynxEyer detector. The source of radiation was the Ka transition of Cu producing radiation with a wavelength λ = 1.54 Å. For recording spectra, the operating current of the instrument was set at 40mA and the voltage at 40 kV. The scan step was 0.02° and the scan rate was 0.25°/min. The range was adjusted from 5° to 40°, the slot of the source was 0.6 mm and the probe slot was 8 mm.

### EXAMPLE 2

### Production of the crystalline dihvdroxvsuccinimidvl salt of N¹,N¹²-di(all-trans-retinoyl)spermine (crRASP)

To a cooled at 0 °C solution of spermine (1.01 g, 5 mmol) in 50 mL anhydrous dichloromethane (DCM) is added the active ester RAOSu (3.78 g, 9.5 mmol). The resulting solution is stirred for 2 hours at 0 °C and room temperature overnight. The precipitated product is filtered, washed on the filter with DCM and diethyl ether and dried under reduced pressure to give 3.79 g (80% yield) of the conjugate RASP in the form of a yellow amorphous fine-grained solid. HPLC chromatogram and X-ray diffraction (XRD) spectrum of RASP are provided in Figures 2 and 3, respectively.

The product (3.79 g) was dissolved in the minimum volume of refluxing methanol (90 mL) under an argon atmosphere, and the resulting solution (if necessary filtered hot through a fluted filter) is allowed to return to room temperature and then stored in the refrigerator (4-8 °C) overnight. The obtained crystalline precipitate is filtered under vacuo, washed on the filter sequentially with ice-cold methanol and diethyl ether, and then dried overnight under vacuo over a suitable desiccant (e.g. P₂O₅). That way, pure crRASP (3.11 g) with m.p. 135.5-138 °C is obtained.

Elemental analysis based on C₅₈H₈₈N₆O₈ [(calculated) found]: C (69.85) 69.66; H (8.89) 8.98; N (8.43) 8.57.

HPLC chromatogram, ESI-MS spectrum, ¹H-NMR and ¹³C-NMR spectra, and X-ray powder diffraction spectrum (XRD) of the product crRASP are provided in Figures 4A, 4B, 5, 6A (and 6B and 6C) and 7, respectively.

### EXAMPLE 3

### Second method of preparation of crystalline dihvdroxvsuccinimidvl salt of N¹,N¹²-di(all-trans-retinoyl)spermine

To a solution of spermine (0.5 g, 2.5 mmol) in 30 mL methanol was added the active ester RAOSu (1.89 g, 4.75 mmol) at room temperature. The reaction mixture is stirred vigorously for 4 hours at the same temperature. The resulting yellow solution is then placed in the refrigerator (4-8 °C) overnight. The precipitated product is filtered, washed with ice-cold methanol and diethyl ether, and dried under reduced pressure to give 0.71 g (30% yield) of crystalline crRASP conjugate.

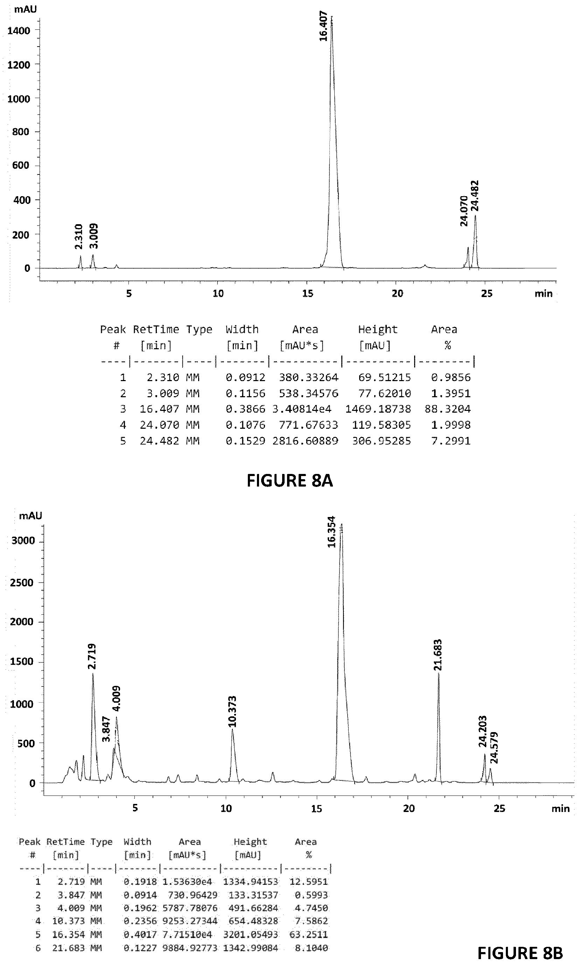

HPLC chromatogram of the crRASP product as obtained by this method is provided in Figure 8A.

### EXAMPLE 4

### Third method for the preparation of crystalline dihydroxysuccinimidyl salt of N¹,N¹²-di(all-trans-retinoyl)spermine (crRASP)

To an ice-cold (0 °C) solution of spermine (1.01 g, 5 mmol) in 50 mL freshly distilled anhydrous chloroform is added the active ester RAOSu (3.78 g, 9.5 mmol). The reaction mixture is stirred for half an hour at 0 °C and half an hour at room temperature, under an argon atmosphere and light exclusion. The resulting yellow solution is evaporated under reduced pressure with the aid of a rotary evaporator and the yellow oily residue thus obtained is dissolved in refluxing methanol (50 mL). The solution is then allowed to attain room temperature and then placed in the refrigerator overnight. The precipitated product is filtered, washed with ice-cold methanol and diethyl ether, and dried under reduced pressure to give 3.32 g (70% yield) of crystalline crRASP.

HPLC chromatogram of the crRASP product as obtained by this method is provided in Figure 9.

### EXAMPLE 5

### Preparation of the crystalline mixed hydrochloride-hydroxysuccinimide salt of N¹,N¹²-di(all-trans-retinoyl)spermine (mcrRASP)

A mixture of RASP or crRASP (2 g, 2 mmol) and NH₄Cl (0.24 g, 4.4 mmol) in MeOH (40 mL) is boiled for 30 minutes. The resulting yellow solution is allowed to come to room temperature and then refrigerated overnight. The precipitated product is filtered, washed on the filter with cold methanol and diethyl ether and dried under reduced pressure to give 1.25 g (68% yield) of the crystalline salt mcrRASP with m.p. 155-157 °C.

Elemental analysis based on C₅₄H₈₄ClN₅O₅ [(calculated) found]: C (70.60) 70.86; H (9.22) 8.97; N (7.62) 7.45.

HPLC chromatogram, ¹H-NMR and ¹³C-NMR spectra, and X-ray powder diffraction spectrum (XRD) of the product mcrRASP are provided in Figures 10, 11, 12A (and 12B and 12C) and 13, respectively.

### EXAMPLE 6

### Solutions crRASP or mcrRASP for oral administration

Examples of solution compositions crRASP and mcrRASP are given in Table 1. To prepare the solutions, all components other than the active substance (crRASP or mcrRASP) and flavoring agents are transferred under stirring to purified water (approximately 80% of the required amount). Once completely dissolved, the active substance (crRASP or mcrRASP) is added under mixing and mild heating. Finally the flavoring agents are added and the final volume of the solution is adjusted by adding pure water.

**Table 1. Examples of solution compositions of crRASP and mcrRASP for oral administration**

| | | **Composition** | | |
|---|---|---|---|---|
| **Ingredient** | **Use** | **A (%)** | **B (%)** | **C (%)** |
| crRASP ή mcrRASP | Active ingredient | 10 | 1 | 1 |
| Ethanol | Co-solvent | 25 | 10 | - |
| Propylene glycol | Co-solvent | 20 | - | 20 |
| Polyethylene glycol (200-600) | Co-solvent | | 30 | 20 |
| Glycerol | Thickening agent (viscosity enhancer) | 10 | - | - |
| Hydroxypropylmethylcellulose | Viscosity enhancer, dissolution aid | - | 2 | |
| Hydroxypropylcellulose | Viscosity enhancer, dissolution aid | - | - | 2 |
| Sorbitol (70%) | Sweetener | 20 | 30 | 30 |
| Sodium saccharin | Sweetener | 0.06 | - | - |
| Acesulfame potassium | Sweetener | 0.04 | - | - |
| Sucralose | Sweetener | - | 0.1 | 0.1 |
| Benzoic acid | Preservative | - | 0.3 | - |
| Methyl paraben | Preservative | - | - | 0.2 |
| Propyl paraben | Preservative | - | - | 0.1 |
| Cherry flavor | flavoring agent | 0.08 | 0.08 | 0.08 |
| Caramel flavor | flavoring agent | 0.02 | 0.02 | 0.02 |
| Purified water | Solvent | q.s. to | q.s. to | q.s. to |
| | | 100 mL | 100 mL | 100 mL |

### EXAMPLE 7

### Solutions crRASP or mcrRASP for parenteral administration

For the preparation of a solution for parenteral administration (intravenous, intramuscular or subcutaneous injection or intravenous infusion), sterile active substance (crRASP or mcrRASP) is dissolved in commercially available sterile aqueous solvents, isotonic to the biological fluids (e.g. isotonic solution of sodium choride or isotonic dextrose solution), under aseptic conditions. Examples of compositions are given in Table 2. Alternatively, the active substance is mixed with a suitable diluent excipient, such as anhydrous lactose, lactose monohydrate, mannitol, glucose, sucrose, trehalose, in an active/excipient ratio of 1:1 to 1:10 w/w, and then it is dissolved in sufficient amount of pure water and lyophilized (Freeze-dried). The lyophilized powder is reconstituted in commercially available sterile aqueous solvents, isotonic with the biological fluids (e.g., isotonic sodium chloride, or isotonic dextrose solution), under aseptic conditions shortly before administration.

**Table 2. Examples of compositions of parenteral solutions of crRASP and mcrRASP**

| | | **Composition (mg)** | | | |
|---|---|---|---|---|---|
| **Ingredient** | **Use** | **A** | **B** | **C** | **D** |
| crRASP or mcrRASP | Active ingredient | 1 | 1 | 5 | 5 |
| Sterile isotonic sodium chloride solution | Solvent | q.s. to 1 ml | - | q.s. to 1 ml | - |
| Sterile isotonic dextrose solution | Solvent | - | q.s. to 1 mL | - | q.s. to 1 mL |

### EXAMPLE 8

### Lotion of crRASP or mcrRASP for topical administration

Table 3 provides examples of lotion formulations of the active ingredient (crRASP or mcrRASP) for topical application. For the preparation of the lotion, the active substance, the oily substance and the perfume are dispersed in ethanol. The remaining ingredients are dispersed in part of the water. Finally, ethanol is added under mixing in the aqueous phase and the volume of water is made up to 100 mL.

**Table 3. Examples of the lotion compositions of crRASP and mcrRASP**

| | | **Composition (%)** | | |
|---|---|---|---|---|
| **Ingredient** | **Use** | **A** | **B** | **C** |
| crRASP or mcrRASP | Active ingredient | 1 | 1 | 1 |
| Ethanol | Co-solvent | 40 | 40 | 20 |
| Propylene glycol | Humectant, sorption enhancer | 5 | 5 | 5 |
| Polyethylene glycol (200-600) | Co-solvent, sorption enhancer | 5 | 5 | 5 |
| Glycerol | Thickening agent (viscosity enhancer) | 5 | 5 | 5 |
| Carboxymethylcellulose sodium | Thickening agent (viscosity enhancer) | - | 2 | 2 |
| Carbomer 980 | Thickening agent (viscosity enhancer) | 0.5 | - | - |
| trilethanolamine | pH regulator | 0.1 | - | - |
| α-tocopherol acetate | Anti-oxidant | 1 | 1 | 1 |
| Safflower oil | Soothing effect on dry / inflammed skin, antioxidant effect | 1 | 1 | - |
| Methyl paraben | Preservative | - | - | 0.3 |
| Propyl paraben | Preservative | - | - | 0.1 |
| Perfume | Odor improvement | 0.2 | 0.2 | 0.2 |
| Purified water | Solvent | q.s. to 100 mL | q.s. to 100 mL | q.s. to 100 mL |

### EXAMPLE 9

### Cream of crRASP or mcrRASP for topical administration

In Table 4, examples are given for cream formulations of the active substance (crRASP or mcrRASP) for topical application. To prepare the cream, the active substance, the perfume and imidazolidinyl urea are dispersed in ethanol. The water-soluble components are dissolved in water under stirring and heating (70-80 °C) and the oily/fat components are mixed under heating (70-80 °C) until a homogeneous mixture is obtained. The aqueous phase is added with stirring to the oily and the emulsion is allowed to cool to 30 °C, whereupon the ethanol solution is added under stirring and the emulsion is allowed to cool to room temperature.

**Table 4. Examples of cream compositions of crRASP and mcrRASP**

| | | **Composition (%)** | | |
|---|---|---|---|---|
| **Ingredient** | **Use** | **A** | **B** | **C** |
| crRASP or mcrRASP | Active ingredient | 1 | 1 | 1 |
| Ethanol | Co-solvent | 5 | 5 | 5 |
| Propylene glycol | Humectant, sorption enhancer | 4 | 2 | 2 |
| Aloe vera gel | Skin conditioner (wound healing) | 0.2 | - | - |
| D- panthenol | Skin protection / anti-inflammatory/anti-irritant agent | - | 6 | 6 |
| (-)α-bisabolol | Skin protection / anti-inflammatory | - | 0.2 | 0.2 |
| α- tocopherol acetate | Anti-oxidant | - | 1 | 1 |
| Paraffin oil | Oily vehicle | 10 | - | - |
| Petrolatum | Oily base | 3 | - | - |
| Stearic alcohol | Oily phase component (viscosity enhancer) | 2 | 2 | - |
| Stearic acid | Oily phase component (in situ emulsifier) | 4 | - | - |
| monostearate glycerol | Emollient/Thickening agent (viscosity enhancer) | - | 3 | 4 |
| Steareth 20 | Oily phase component (Emulsifier) | 2 | - | - |
| Ceteareth 6 | Oily phase component (Emulsifier) | - | 5 | 5 |
| Ceteareth 25 | Oily phase component (Emulsifier | - | 1 | 1 |
| Polysorbate 60 | Emulsifier | 2 | - | - |
| Dimethicone | Skin protection / anti-foaming | 1 | 1 | 1 |
| polyethylene glycol (150) stearrate | Oily phase component (Emulsifier) | 2 | - | - |
| Isopropanol myristate | Emollient | - | 4 | 2 |
| Lanolin alcohols | Emollient | 1 | - | 1 |
| BHA | Anti-oxidant | 0.1 | - | - |
| Imidazolidinyl-urea | Preservative | 0.2 | - | - |
| Methyl paraben | Preservative | 0.2 | 0.4 | 0.4 |
| Propyl paraben | Preservative | 0.1 | 0.2 | 0.2 |
| Perfume | Odor improvement | 0.15 | 0.15 | 0.15 |
| Sodium borate | pH regulator | 0.2 | - | - |
| Purified | Solvent | 61,85 | 69,05 | 71,05 |

### EXAMPLE 10

### Tablets of crRASP or mcrRASP for oral administration

Examples of tablet compositions crRASP and mcrRASP are given in Table 5. The tablets of formulation A are produced by direct compression whereas the tablets of composition B are produced by tableting after wet granulation.

**Table 5. Examples of tablet compositions of crRASP and mcrRASP for oral administration**

| **Ingredient** | **Use** | **Composition (%)** | |
|---|---|---|---|
| | | **A** | **B** |
| crRASP or mcrRASP | Active ingredient | 10 | 10 |
| Microcrystalline cellulose | Diluent/ Binder | 60 | 21 |
| Lactose monohydrate | Diluent | 24 | 60 |
| Povidone (PVP) | Binder | - | 2 |
| Sodium salt of carboxymethyl starch (Primojel, Explotab) | Disintegrant | 3 | - |
| Povidone cross-linked (Polyplasdone XL, Crosspovidone) | Disintegrant | | 5 |
| Colloidal silicon dioxide (Cab-O Sil, Syloid, Aerosil | Glidant | 2 | 1 |
| Magnesium stearate | Lubricant | 1 | 1 |
| Purified water* | Solvent | - | q.s. |

| | | | |
|---|---|---|---|
| * Does not remain in the finished product | | | |

## Claims

1. A process for the production of a crystalline dihydroxysuccinimidyl salt of *N*¹,*N*¹²-di(*all*-*trans*-retinoyl)spermine (crRASP), which comprises the step of crystallization in the presence of an organic solvent.

2. A process according to claim 1, in which the crystallization solvent is an alcohol with a boiling point lower than 100 °C, preferably methanol.

3. A process according to claim 2, comprising the steps of a) reacting spermine with the succinimidyl ester of all-trans-retinoic acid in the solvent dichloromethane, b) filtration and (c) crystallization of the thus obtained crude dihydroxysuccinimidyl salt of *N*¹,*N*¹²-di(*all*-*trans*-retinoyl)spermine in the presence of an alcohol with a boiling point lower than 100 °C, preferably methanol, thereby producing the crystalline dihydroxysuccinimidyl salt of *N*¹,*N*¹²-di(*all-trans*-retinoyl)spermine (crRASP) with a purity equal to or over 98%.

4. A process according to claim 2, comprising the steps of a) reacting spermine with the succinimidyl ester of *all-trans*-retinoic acid in the solvent chloroform, b) evaporation of the solvent under reduced pressure, and (c) crystallization of the thus obtained crude dihydroxysuccinimidyl salt of *N*¹,*N*¹²-di(*all-trans*-retinoyl)spermine in the presence of an alcohol with a boiling point lower than 100 °C, preferably methanol, thereby producing the crystalline dihydroxysuccinimidyl salt of *N*¹*,N*¹²-di(*all-trans-*retinoyl)spermine (crRASP) with a purity equal to or over 92%.

5. A process for the production of a crystalline mixed hydrochloride-hydroxysuccinimide salt of *N*¹,*N*¹²-di(*all*-*trans*-retinoyl)spermine (mcrRASP), which comprises the steps of a) reacting the dihydroxysuccinimidyl salt of *N*¹*,N*¹²*-*di(*all-trans-*retinoyl)spermine (RASP) with ammonium chloride at the refluxing temperature of a solvent, said solvent being an alcohol with a boiling point lower than 100 °C, preferably methanol, and b) the direct crystallization from this solvent.

6. A process for the production of a crystalline mixed hydrochloride-hydroxysuccinimide salt of *N*¹,*N*¹²-di(*all-trans*-retinoyl)spermine (mcrRASP) which comprises the steps of a) reacting the crystalline dihydroxysuccinimidyl salt of *N*¹,*N*¹²-di(*all*-*trans*-retinoyl)spermine (crRASP) with ammonium chloride at the refluxing temperature of a solvent, said solvent being an alcohol with a boiling point lower than 100 °C, preferably methanol, and b) the direct crystallization from this solvent.

7. A crystalline form of the dihydroxysuccinimidyl salt of *N*¹*,N*¹²*-*di(*all-trans-*retinoyl)spermine (crRASP), which is characterized from a powder X-ray diffraction spectrum (XRD) with main peaks at 11.43, 13.33, 16.11, 19.44, 22.40 and 22.72 ±0.20 degrees 2θ.

8. A crystalline form of the mixed hydrochloride-hydroxysuccinimide salt of *N*¹,*N*¹²-di(*all*-*trans*-retinoyl)spermine (mcrRASP), which is characterized from a powder X ray diffraction spectrum (XRD) with main peaks at 9.02, 11.29, 11.39, 13.49, 17.92, 22.36, 22.66, 23.00, 23.62, 23.76, 23.84, 24.42, 24.58 and 25.38 ±0.20 degrees 2θ.

9. The crystalline dihydroxysuccinimidyl salt of *N*¹,*N*¹²-di(*all-trans*-retinoyl)spermine (crRASP) according to claim 7, or the crystalline mixed hydrochloride-hydroxysuccinimide salt of *N*¹,*N*¹²-di(*all-trans*-retinoyl)spermine (mcrRASP) according to claim 8, for use in medicine.

10. The crystalline dihydroxysuccinimidyl salt of *N*¹,*N*¹²-di(*all-trans*-retinoyl)spermine (crRASP) according to claim 7, or the crystalline mixed hydrochloride-hydroxysuccinimide salt of *N*¹,*N*¹²-di(*all-trans*-retinoyl)spermine (mcrRASP) according to claim 8, for use in the treatment of dermatological diseases, such psoriasis, skin inflammation and acne.

11. Pharmaceutical compositions comprising the crystalline dihydroxysuccinimidyl salt of *N*¹,*N*¹²-di(*all-trans*-retinoyl)spermine (crRASP) according to claim 7, or the crystalline mixed hydrochloride-hydroxysuccinimide salt of *N*¹*,N*¹²*-*di(a*ll-trans-*retinoyl)spermine (mcrRASP) according to claim 8, for use in medicine.

12. Pharmaceutical compositions comprising the crystalline dihydroxysuccinimidyl salt of *N*¹,*N*¹²-di(*all-trans*-retinoyl)spermine (crRASP) according to claim 7, or the crystalline mixed hydrochloride-hydroxysuccinimide salt of *N*¹*,N*¹²*-*di(*all-trans-*retinoyl)spermine (mcrRASP) according to claim 8, for use in the treatment of dermatological diseases such as psoriasis, skin inflammation and acne.

13. Pharmaceutical compositions comprising the crRASP or the mcrRASP for use according to claim 11 or 12, for topical, parenteral or oral administration thereof.

14. Use of the crystalline dihydroxysuccinimidyl salt of *N*¹*,N*¹²*-*di(*all-trans-*retinoyl)spermine (crRASP) or the crystalline mixed hydrochloride-hydroxysuccinimide salt of *N*¹,*N*¹²-di(*all-trans*-retinoyl)spermine (mcrRASP) for the production of cosmetic compositions for the care and maintenance of good skin condition and/or anti-aging, wherein said formulations optionally comprise suitable excipients for cosmetic uses.
